# EUROPÄISCHE PATENTSCHRIFT

(11) **EP 2 729 451 B1**
(45) Veröffentlichungstag und Bekanntmachung des Hinweises auf die Patenterteilung: **22.06.2016**
(21) Anmeldenummer: 12738052.5
(22) Anmeldetag: 05.07.2012
(51) Int. Cl.: C07D 233/32, C07D 233/34, C07C 67/03, C07C 67/08, C07C 69/54

(54) **VERFAHREN ZUR HERSTELLUNG VON (METH)ACRYLSÄUREESTERN**
METHOD FOR PRODUCING (METH)ACRYLIC ACID ESTERS
PROCEDE DE PRODUCTION D'ESTERS D'ACIDE (MÉTH)ACRYLIQUE

(30) Priorität: 06.07.2011 EP 11172882
(43) Veröffentlichungstag der Anmeldung: 14.05.2014
(73) Patentinhaber: BASF SE, 67056 Ludwigshafen am Rhein (DE)
(72) Erfinder: BETTE, Virginie, 68199 Mannheim (DE); DUST, Matthias, 67071 Ludwigshafen (DE); PETZOLDT, Jochen, 67273 Weisenheim am Berg (DE); MEISENBURG, Uwe, 68219 Mannheim (DE); MISSKE, Andrea, 67346 Speyer (DE); BERGMANN, Hermann, 68165 Mannheim (DE)
(74) Vertreter: BASF IP Association
(86) Internationale Anmeldenummer: PCT/EP2012/063073
(87) Internationale Veröffentlichungsnummer: WO 2013/004767

(56) Entgegenhaltungen:
- EP-A1- 1 574 533
- EP-A1- 1 686 118

## Beschreibung

Die vorliegende Erfindung betrifft ein Verfahren zur Herstellung von (Meth)acrylsäureestern durch Veresterung von (Meth)acrylsäure oder Umesterung von mindestens einem (Meth)acrylsäureester mit mindestens einer Verbindung, die mindestens eine OH-Gruppe enthält, in Gegenwart eines heterogenen Katalysators, der mindestens ein anorganisches Salz enthält. Die Erfindung betrifft ferner die Verwendung der hergestellten (Meth)acrylsäureester.

Unter (Meth)acrylsäure im Sinne der vorliegenden Erfindung werden Acrylsäure und/oder Methacrylsäure verstanden, unter (Meth)acrylsäureester Acrylsäureester und/oder Methacrylsäureester. Im Folgenden werden (Meth)acrylsäureester auch als (Meth)acrylate bezeichnet.

Strahlungshärtbare Bindemittel auf Basis von monomeren und oligomeren (Meth)acrylaten haben durch ihre lösungsmittelfreie und leichte Verarbeitbarkeit ein zunehmendes Marktinteresse als Lackharze gefunden, die insbesondere für Dispersionen eingesetzt werden. Daher besteht an verbesserten Herstellungsverfahren für (Meth)acrylate ein anhaltender Bedarf.

Die Herstellung von (Meth)acrylaten erfolgt zumeist durch katalytische Veresterung von (Meth)acrylsäure oder Umesterung von anderen (Meth)acrylsäureestern mit Alkoholen. Das Reaktionsprodukt enthält typischerweise das gewünschte (Meth)acrylat in der als Edukt eingesetzten (Meth)acrylsäure beziehungsweise dem als Edukt eingesetzten (Meth)acrylsäureester und gegebenenfalls alkoholischen Nebenprodukten.

Zur Herstellung von (Meth)acrylsäureestern können unterschiedliche Katalysatoren eingesetzt werden. Aus DE 27 44 641 A1 beispielsweise ist Lithiumhydroxid als Katalysator bekannt. Alkalimetalle und Alkalimetallhydroxide hydrolysieren jedoch schon bei geringem Wassergehalt und erfordern daher wasserfreie Reaktionsbedingungen. EP-A 1 236 994 beschreibt die Herstellung von Ureido(meth)acrylaten durch Umesterung von (Meth)acrylat in Gegenwart von Titanalkoholaten oder 1,3-Dicarbonylchelaten von Titan, Zirkonium, Eisen oder Zink.

Nachteilig an diesen Verbindungen ist, dass die Metallverbindungen feuchtigkeitsempfindlich sind und daher leicht desaktivieren. Zudem beeinflussen im Produkt verbleibende Spuren der Katalysatoren eine eventuell nachfolgende Polymerisation, und müssen daher aufwändig aus dem Produkt entfernt werden. Eine solche Entfernung wird zumeist mittels einer wässrigen Wäsche durchgeführt, so dass das Produkt anschließend getrocknet werden muss.

WO 2006/012980 beschreibt die Herstellung von (Meth)acrylaten von ringförmigen oder offenkettigen N-hydroxyalkylierten Amiden, wobei in Gegenwart eines heterogenen Katalysators aus anorganischen Salzen (Meth)acrylsäure verestert oder mindestens ein (Meth)acrylsäureester umgeestert wird. Als anorganisches Salz wird unter anderem K₃PO₄ in wasserfreier Form eingesetzt. Nachteilig daran ist, dass wasserfreies K₃PO₄ sehr schnell unter der Bildung von Nebenprodukten reagiert.

US 7,528,278 beschreibt ein Umesterungsverfahren, das als heterogenen Katalysator eine Mischung aus anorganischen Salzen einsetzt, wobei das Umesterungsverfahren insbesondere in Gegenwart einer Mischung aus Kaliumcarbonat und Kaliumchlorid durchgeführt wird. Unter Verwendung dieses Katalysators kann die Reaktion bis zu einem Wasseranteil von 3000 ppm bezogen auf das Gesamtgewicht des Reaktionsgemisches noch hinreichende Umsätze liefern. Allerdings kann die Umsetzung mit Kaliumcarbonat wesentlich langsamer verlaufen als mit Kaliumpllosphat.

EP 1 686 118 A1 beschreibt ein Verfahren zur Herstellung von (Meth)acrylsäureestern durch Umestern eines (Meth)acrylsäureesters mit Alkohol in Gegenwart eines Katalysators. Der Reaktionsmischung, der durch einen Dehydrierungsschritt Wasser entzogen wird, wird der Katalysator zugesetzt, welcher bei den Reaktionsbedingungen vollständig gelöst ist und so als homogener Katalysator wirkt.

Trotz des umfangreichen Standes der Technik auf dem Gebiet der Herstellung von (Meth)acrylaten besteht ständiger Optimierungsbedarf, um die chemischen Prozesse durch höhere Ausbeuten effizienter zu gestalten und gleichzeitig helle Veresterungsprodukte bereitzustellen, die beispielsweise in Dispersionen Verwendung finden.

Aufgabe der vorliegenden Erfindung ist es, ein einfaches Verfahren zur Verfügung zu stellen, mit dem helle Veresterungsprodukte in guter Ausbeute herstellbar sind. Insbesondere soll der Anteil von mehrfach (meth)acrylierten Produkten zurückgedrängt werden.

Die Aufgabe wird gelöst durch ein Verfahren zur Herstellung von (Meth)acrylsäureestern durch Veresterung von (Meth)acrylsäure oder Umesterung von mindestens einem (Meth)acrylsäureester mit mindestens einer Verbindung, die mindestens eine OH-Gruppe enthält, in Gegenwart eines heterogenen Katalysators, der mindestens ein anorganisches Salz, ausgewählt aus der Gruppe bestehend aus K₃PO₄, Na₃PO₄, K₂CO₃ und Na₂CO₃ sowie deren Hydrate, umfasst, wobei die Veresterung oder die Umesterung in Gegenwart von 300 bis 3000 ppm Wasser bezogen auf das Gesamtgewicht des Reaktionsgemisches durchgeführt wird.

Überraschenderweise wurde gefunden, dass bei der Herstellung von (Meth)acrylaten die Zugabe der erfindungsgemäßen Menge von Wasser die Selektivität erhöht. Zusätzlich wird durch das erfindungsgemäße Verfahren die Polymerbildung verringert und die erhaltenen Veresterungsprodukte weisen eine gute Farbzahl auf.

Im Rahmen der Erfindung bedeutet ppm bezogen auf das Gesamtgewicht des Reaktionsgemisches einen Massenteil in einer Million und kann in Milligramm pro Kilogramm angegeben werden. Hierbei umfasst das Reaktionsgemisch sowohl die Reaktanden als auch etwaige Hilfsmittel wie beispielsweise Lösungsmittel oder Polymerisationsinhibitoren. Im Folgenden ist ppm falls nicht anders spezifiziert, als ppm bezogen auf das Gesamtgewicht des Reaktionsgemisches zu verstehen.

Erfindungsgemäß wird die Veresterung oder die Umesterung in Gegenwart von 300 bis 3000 ppm Wasser, vorzugsweise 400 bis 2000 ppm Wasser durchgeführt. Besonders bevorzugt sind 500 bis 1200 ppm Wasser, zum Beispiel 800 ppm.

Im Rahmen dieser Schrift enthält Wasser im Wesentlichen H₂O und liegt vorzugsweise im flüssigen Aggregatzustand vor. Dabei bedeutet im Wesentlichen H₂O, dass das Wasser mindestens 95% (bezogen auf das Gewicht des Wassers), vorzugsweise mindestens 99% (bezogen auf das Gewicht des Wassers) H₂O enthält.

Das Wasser kann zusätzlich einen üblichen Anteil an Unreinheiten enthalten, der kleiner 5% (bezogen auf das Gewicht des Wassers), bevorzugt kleiner 1%, sein kann. Als Unreinheiten können ein oder mehrere der folgenden Verbindungen in ungelöster oder gelöster Form in dem Wasser vorliegen:
- anorganische Verbindungen, zum Beispiel Salze und Mineralien, die beispielhaft folgende Ionen enthalten können:
   - Kationen: Natrium, Kalium, Magnesium, Calcium, Eisen, Aluminium;
   - Anionen: Chlorid, lodid, Bromid, Sulfit, Sulfid, Sulfat, Carbonat, Fluorid, Phosphat, Silikat, Nitrat, Nitrit;
- organische Verbindungen und
- Mikroorganismen.

Beim Ansetzen des Reaktionsgemisches kann das Wasser in einem beliebigen Verfahrensschritt vollständig beigefügt werden. Alternativ kann die Zugabe von Wasser in Teilen in mehreren Verfahrensschritten erfolgen, so dass in der Summe die gewünschte Gesamtmenge an Wasser vorliegt. Typischerweise werden in einem ersten Verfahrensschritt beim Ansetzen des Reaktionsgemisches (Meth)acrylsäure oder mindestens ein (Meth)acrylsäureester sowie gegebenenfalls Polymerisationsinhibitoren und gegebenenfalls Lösungsmittel vorgelegt. In einem nächsten Verfahrensschritt kann diese Mischung falls erforderlich aufgeheizt werden. Darauf folgt die Zugabe der mindestens eine OH-Gruppe enthaltenden Verbindungen und des heterogenen Katalysators. Diese Mischung bildet den Ansatz zur Veresterung von (Meth)acrylsäure oder Umesterung von mindestens einem (Meth)acrylsäureester.

Das Wasser kann vollständig oder in Teilen in einem Verfahrensschritt vor oder nach Zugabe des heterogenen Katalysators zugeführt werden. Bevorzugt ist die vollständige Zugabe des Wassers vor der Zugabe des heterogenen Katalysators, besonders bevorzugt vor der Zugabe des Alkohols.

Die erfindungsgemäß eingesetzten Katalysatoren zeigen eine geringe Tendenz unter dem Einfluss der erfindungsgemäß bestimmten Wassermenge zu desaktivieren. Dazu eignen sich insbesondere anorganische Salze, die ausreichend basisch sind, um eine Ver- oder Umesterung zu katalysieren, und in Kombination mit der erfindungsgemäßen Wassermenge Nebenreaktionen wie beispielsweise die Michael-Reaktion zurückdrängen.

Der heterogene Katalysator kann in wasserfreier Form vorliegen. Auf diese Weise kann eine präzise Dosierung des Wassers erreicht werden. Alternativ kann ein Teil des Wassers in dem heterogenen Katalysator enthalten sein und dieser Teil in dem heterogenen Katalysator beispielsweise als Kristallwasser gebunden sein kann. Der in dem heterogenen Katalysator enthaltene Teil des Wassers kann bei der Dosierung der Wassermenge berücksichtigt werden.

Der heterogene Katalysator kann bezogen auf das Gesamtgewicht des Katalysators bis zu 10 % Wasser enthalten.

Heterogene Katalysatoren sind im Rahmen der Erfindung solche, die eine Löslichkeit im Reaktionsgemisch bei 25 °C von nicht mehr als 1 g/l aufweisen, bevorzugt von nicht mehr als 0,5 g/l und besonders bevorzugt von nicht mehr als 0,25 g/l.

Erfindungsgemäß einsetzbare anorganische Salze sind solche, die einen pK_{B}-Wert von nicht mehr als 7,0, bevorzugt von nicht mehr als 6,1 und besonders bevorzugt von nicht mehr als 4,0 aufweisen. Gleichzeitig sollte der pK_{B}-Wert 1,0 nicht unterschreiten, bevorzugt nicht weniger als 1,5 betragen und besonders bevorzugt nicht weniger als 1,6.

Das anorganische Salz weist bevorzugt mindestens ein Anion auf ausgewählt aus der Gruppe bestehend aus Carbonat (CO₃²⁻), Hydrogencarbonat (HCO₃⁻), Phosphat (PO₄³⁻) Hydrogenphosphat (HPO₄²⁻), Dihydrogenphosphat (H₂PO₄⁻), Sulfat (SO₄²⁻), Sulfit (SO₃²⁻) und Carboxylat (RCOO⁻), worin R C₁-C₁₈-Alkyl oder gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl oder C₆-C₁₂-Aryl bedeutet.

Bevorzugt sind Carbonat und Phosphat, besonders bevorzugt ist Phosphat.

Unter Phosphat sind auch die Kondensationsprodukte zu verstehen, wie beispielsweise Diphosphate, Triphosphate und Polyphosphate.

Das anorganische Salz weist bevorzugt mindestens ein Kation auf ausgewählt aus der Gruppe bestehend aus Alkalimetallen, Erdalkalimetallen, Ammonium, Cer, Eisen, Mangan, Chrom, Molybdän, Kobalt, Nickel oder Zink.

Bevorzugte Alkalimetalle sind Natrium oder Kalium.

Besonders bevorzugte anorganische Salze sind K₃PO₄, Na₃PO₄, K₂CO₃ und Na₂CO₃ sowie deren Hydrate, ganz besonders bevorzugt ist K₃PO₄.

(Meth)acrylate können (Meth)acrylsäure oder Ester von (Meth)acrylsäure mit einem gesättigten Alkohol sein, bevorzugt gesättigte C₁-C₁₀-Alkylester der (Meth)acrylsäure, besonders bevorzugt gesättigte C₁-C₄-Alkylester der (Meth)acrylsäure.

Gesättigt bedeutet im Rahmen der Erfindung Verbindungen ohne C-C-Mehrfachbindungen (außer selbstverständlich die C=C-Doppelbindung in den (Meth)acryleinheiten).

Beispiele für Verbindungen sind (Meth)acrylsäuremethyl-, -ethyl-, -n-butyl-, -*iso*-butyl-, -*tert*-butyl-, n-octyl- und -2-Ethylhexylester, 1,2-Ethylenglykoldi- und -mono(meth)-acrylat, 1,4-Butandioldi- und -mono(meth)acrylat, 1,6-Hexandioldi- und -mono(meth)-acrylat, Trimethylolpropantri(meth)acrylat und Pentaerythrittetra(meth)acrylat.

Besonders bevorzugt sind (Meth)acrylsäuremethyl-, -ethyl-, -n-butyt- und -2-Ethylhexylester, ganz besonders bevorzugt (Meth)acrylsäuremethyl-, -ethyl- und -n-butylester, insbesondere (Meth)acrylsäuremethyl- und -ethylester und speziell (Meth)acrylsäuremethylester.

Als Verbindungen mit einer oder mehreren OH-Gruppe(n) kommen insbesondere N-hydroxyalkylierte Amide in Betracht. N-hydroxyalkylierte Amide im Sinne der Erfindung sind solche Verbindungen, die mindestens eine Z¹-(C=O)-N-Gruppe und mindestens eine Hydroxygruppe (-OH) aufweisen, wobei die Hydroxygruppe mit dem Stickstoffatom der Z'-(C=O)-N-Gruppe verbunden ist und Z¹ definiert ist wie unten aufgeführt. Die N-hydroxyalkylierten Amide können ringförmige (C) N-hydroxyalkylierte Amide 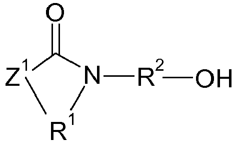 oder offenkettige (O) N-hydroxyalkylierte Amide 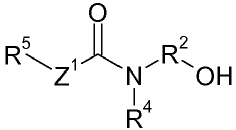 sein.

In diesem Zusammenhang bedeuten:
Z¹ Sauerstoff, Schwefel, unsubstituierter oder substituierter Phosphor oder un- oder monosubstituierter Stickstoff (N-R³),
R¹ und R² jeweils unabhängig voneinander C₂-C₂₀-Alkylen, C₅-C₁₂-Cycloakylen, C₆-C₁₂-Arylen oder durch ein oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können, oder
R²-OH für eine Gruppe der Formel -[Xᵢ]ₖ-H,
R³, R⁴ und R⁵ unabhängig voneinander jeweils Wasserstoff, C₁-C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂-C₁₈- Alkyl, C₂-C₁₈-Alkenyl, C₆-C₁₂-Aryl, C₅-C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, wobei R³ und R⁵ auch gemeinsam einen fünfbis zwölfgliedrigen Ring bilden können,
mit der Maßgabe, dass im Fall von Z¹ = O R⁵ lediglich unsubstituiertes C₁-C₁₈-Alkyl, C₆-C₁₂-Aryl oder C₅-C₁₂-Cycloalkyl ist,
k für eine Zahl von 1 bis 50 und
Xᵢ für jedes i = 1 bis k unabhängig voneinander ausgewählt sein kann aus der Gruppe -CH₂-CH₂-O-, -CH₂-CH₂-N(H)-, -CH₂-CH₂-CH₂-N(H)-, -CH₂-CH(NH₂)-, -CH₂-CH(NHCHO)-, -CH₂-CH(CH₃)-O-, -CH(CH₃)-CH₂-O-, -CH₂-C(CH₃)₂-O-, -C(CH₃)₂-CH₂-O-, -CH₂-CH₂-CH₂-O-, -CH₂-CH₂-CH₂- CH₂-O-, -CH₂-CHVin-O-, -CHVin-CH₂-O-, -CH₂-CHPh-O und -CHPh-CH₂-O-, worin Ph für Phenyl und Vin für Vinyl steht.

In den obigen Definitionen bedeuten
gegebenenfalls durch Aryl, Alkyl, Aryloxy, Alkyloxy, Heteroatome und/oder Heterocyclen substituiertes C₂-C₂₀-Alkylen beispielsweise 1,2-Ethylen, 1,2-Propylen, 1,3-Propylen, 1,4-Butylen, 1,6-Hexylen, 2-Methyl-1,3-propylen, 2-Ethyl-1,3-propylen, 2,2- Dimethyl-1,3-propylen, 2,2-Dimethy-1,4-butylen,
gegebenenfalls durch Aryl, Alkyl, Aryloxy, Alkyloxy, Heteroatome und/oder Heterocyclen substituiertes C₅-C₁₂-Cycloalkylen beispielsweise Cyclopropylen, Cyclopentylen, Cyclohexylen, Cyclooctylen, Cyclododecylen,
gegebenenfalls durch Aryl, Alkyl, Aryloxy, Alkyloxy, Heteroatome und/oder Heterocyclen substituiertes, durch ein oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen beispielsweise 1-Oxa-1,3-propylen, 1,4-Dioxa-1,6-hexylen, 1,4,7-Trioxa-1,9-nonylen, 1-Oxa-1,4-butylen, 1,5-Dioxa-1,8-octylen, 1-Oxa-1,5-pentylen, 1-Oxa-1,7-heptylen, 1,6-Dioxa-1,10-decylen, 1-Oxa-3-methyl-1,3-propylen, 1-Oxa-3-methyl-1,4-butylen, 1-Oxa-3,3-dimethyl-1,4-butylen, 1-Oxa-3,3-dimethyl-1,5-pentylen, 1,4-Dioxa-3,6-dimethyl-1,6-hexylen, 1-Oxa-2-methyl-1,3-propylen, 1,4-Dioxa-2,5-dimethyl-1,6-hexylen, 1-Oxa-1,5-pent-3-enylen, 1-Oxa-1,5-pent-3-inylen, 1,1-, 1,2-, 1,3- oder 1,4-Cyclohexylen, 1,2- oder 1,3-Cyclopentylen, 1,2-, 1,3- oder 1,4-Phenylen, 4,4'-Biphenylen, 1,4-Diaza-1,4-butylen, 1-Aza-1,3-propylen, 1,4,7-Triaza-1,7-heptylen, 1,4-Diaza-1,6-hexylen, 1,4-Diaza-7-oxa-1,7-heptylen, 4,7-Diaza-1-oxa-1,7-heptylen, 4-Aza-1-oxa-1,6-hexylen, 1-Aza-4-oxa-1,4-butylen, 1-Aza-1,3-propylen, 4-Aza-1-oxa-1,4-butylen, 4-Aza-1,7-dioxa-1,7-heptylen, 4-Aza-1-oxa-4-methyl-1,6-hexylen, 4-Aza-1,7-dioxa-4-methyl-1,7-heptylen, 4-Aza-1,7-dioxa-4-(2'-hydroxyethyl)-1,7-heptylen, 4-Aza-1-oxa-(2'-hydroxyethyl)-1,6-hexylen oder 1,4-Piperazinylen,
gegebenenfalls durch Aryl, Alkyl, Aryloxy, Alkyloxy, Heteroatome und/oder Heterocyclen substituiertes C₆-C₁₂-Arylen beispielsweise 1,2-, 1,3- oder 1,4-Phenylen, 4,4'-Biphenylen, Toluylen oder Xylylen,
C₁-C₁₈-Alkyl oder gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl, beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Hexadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl, 1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonylethyl, 2-Ethoxycarbonylethyl, 2-Butoxycarbonylpropyl, 1,2-Di(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl,
gegebenenfalls durch ein oder mehrere Sauerstoff- und/oder Schwefelatome und/oder ein oder mehrere substituierte oder unsubstituierte Iminogruppen unterbrochenes C₂-C₁₈-Alkenyl beispielsweise Vinyl, 1-Propenyl, Allyl, Methallyl, 1,1-Dimethylallyl, 2-Butenyl, 2-Hexenyl, Octenyl, Undecenyl, Dodecenyl, Octadecenyl, 2-Phenylvinyl, 2-Methoxyvinyl, 2-Ethoxyvinyl, 2-Methoxyallyl, 3-Methoxyallyl, 2-Ethoxyallyl, 3-Ethoxyallyl oder 1- oder 2-Chlorvinyl,
gegebenenfalls durch ein oder mehrere Sauerstoff- und/oder Schwefelatome und/oder ein oder mehrere substituierte oder unsubstituierte Iminogruppen unterbrochenes C₆-C₁₂-Aryl beispielsweise Phenyl, Tolyl, Xylyl, α-Naphthyl, β-Naphthyl, 4-Diphenyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl,
gegebenenfalls durch ein oder mehrere Sauerstoff und/oder Schwefelatome und/oder ein oder mehrere substituierte oder unsubstituierte Iminogruppen unterbrochenes C₅-C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl,
und
gegebenenfalls durch ein oder mehrere Sauerstoff- und/oder Schwefelatome und/oder ein oder mehrere substituierte oder unsubstituierte Iminogruppen unterbrochenes fünfbis sechsgliedrigen, Sauerstoff-, Stickstoff und/oder Schwefelatome aufweisenden Heterocyclus, beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl.

Beispiele für R¹ sind 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,6-Hexylen, 2-Methyl-1,3-propylen, 2-Ethyl-1,3-propylen, 2,2-Dimethyl-1,3-propylen und 2,2-Dimethyl-1,4-butylen, 1,2-Cyclopentylen, 1,3-Cyclopentylen, 1,2-Cyclohexylen,1,3- Cyclohexylen und ortho-Phenylen. Bevorzugt sind 1,2-Ethylen, 1,2-Propylen, 1,3-Propylen, besonders bevorzugt sind 1,2-Ethylen und 1,2-Propylen und ganz besonders bevorzugt ist 1,2-Ethylen.

Beispiele für R² sind 1,2-Ethylen, 1,2-Propylen, 1,1-Dimethyl-1,2-ethylen, 1-Hydroxymethyl-1,2-ethylen, 2-Hydroxy-1,3-propylen, 1,3-Propylen, 1,4-Butylen, 1,6-Hexylen, 2-Methyl-1,3-propylen, 2-Ethyl-1,3-propylen, 2,2-Dimethyl-1,3-propylen und 2,2-Dimethyl-1,4-butylen, 1,2-Cyclopentylen, 1,3-Cyclopentylen, 1,2-Cyclohexylen,1,3-Cyclohexylen und ortho-Phenylen. Bevorzugt sind 1,2-Ethylen, 1,2-Propylen, 1,3-Propylen, besonders bevorzugt sind 1,2-Ethylen und 1,2-Propylen und ganz besonders bevorzugt ist 1,2-Ethylen.

Beispiele für R³ und R⁵ sind unabhängig voneinander Wasserstoff oder C₁-C₄-Alkyl, was im Rahmen der vorliegenden Erfindung für Methyl, Ethyl, *iso*-Propyl, n-Propyl, n-Butyl, *iso*-Butyl, sec-Butyl oder *ter*t-Butyl steht, bevorzugt für Wasserstoff, Methyl, Ethyl, n-Propyl und n-Butyl, besonders bevorzugt für Wasserstoff, Methyl, Ethyl und n-Butyl und ganz besonders bevorzugt für Wasserstoff, Methyl und Ethyl und insbesondere für Wasserstoff.

Wenn R³ und R⁵ einen gemeinsamen Ring bilden, so können R³ und R⁵ gemeinsam 1,4-Butylen, 1,5-Pentylen oder 3-Oxa-1,5-pentylen bedeuten.

Beispiele für R⁴ sind Wasserstoff, Methyl, Ethyl, *iso*-Propyl, n-Propyl, n-Butyl, *iso*-Butyl, sec-Butyl, *tert*-Butyl, n-Hexyl, n-Heptyl, n-Octyl, n-Decyl, n-Dodecyl, n-Tetradecyl, n-Hexadecyl, n-Octadecyl, n-Eicosyl, 2-Ethylhexyl, Cyclopentyl, Cyclohexyl, Cyclooctyl, Cyclododecyl, Phenyl, Naphthyl oder Benzyl.

Z¹ ist bevorzugt O oder NR³, besonders bevorzugt NR³.

Bevorzugt sind die ringförmigen Amide (C) gegenüber den offenkettigen (O) Amiden.

Bevorzugte ringförmige Amide (C) sind 2'-Hydroxyethylethylenharnstoff, 3'-Hydroxypropylethylenharnstoff, 2'-Hydroxypropylethylenharnstoff, 2'-Hydroxyethyl-1,3-propylenharnstoff, 2'-Hydroxypropyl-1,3-propylenharnstoff, 3'-Hydroxypropyl-1,3-propylenharnstoff, 2'-Hydroxyethyl-1,2-propylenharnstoff, 2'-Hydroxypropyl-1,2-propylenharnstoff, 3'-Hydroxypropyl-1,2-propylenharnstoff, 1-(2'-Hydroxyethyl)-imidazolidin-2,4-dion, 1-(2'-Hydroxyethyl)-dihydropyrimidin-2,4-dion oder 3-(2-Hydroxyethyl)-oxazolidin-2-on. Besonders bevorzugt sind 2'-Hydroxyethylethylenharnstoff, 3'-Hydroxypropylethylenharnstoff, 2'-Hydroxyethyl-1,3-propylenharnstoff und 3'-Hydroxypropyl-1,3-propylenharnstoff.

Bevorzugte offenkettige Amide sind N-(2-Hydroxyethyl)harnstoff, N-(2-Hydroxypropyl)harnstoff, N-(3-Hydroxypropyl)harnstoff, N',N'-Dimethyl-N-(2-hydroxyethyl)harnstoff, N',N'-Dimethyl-N-(2-hydroxypropyl)harnstoff, N',N'-Dimethyl-N(3-hydroxypropyl)harnstoff, N,N'-Diethyl-N-(2-hydroxyethyl)harnstoff, N',N'-Diethyl-N(2-hydroxypropyl)harnstoff, N',N'-Diethyl-N-(3-hydroxypropyl)harnstoff, N',N'-Di-n-butyl-N-(2-Hydroxyethyl)harnstoff, N',N'-Di-n-butyl-N-(2-Hydroxypropyl)harnstoff und N',N'-Di-n-butyl-N-(3-hydroxypropyl)harnstoff, besonders bevorzugt sind N-(2-Hydroxyethyl)harnstoff, N-(2-Hydroxypropyl)harnstoff und N-(3-Hydroxypropyl)harnstoff.

Die Veresterung mit (Meth)acrylsäure oder bevorzugt die Umesterung des Amids (C) oder (O) erfolgt mit mindestens einem, bevorzugt einem (Meth)acrylat in Anwesenheit mindestens eines heterogenen Katalysators umfassend mindestens ein anorganisches Salz, bevorzugt ein anorganisches Salz.

Besonders bevorzugt eignet sich das erfindungsgemäße Verfahren zur Herstellung von Ureido(meth)acrylaten durch Umesterung von (Meth)acrylat. Ureidoalkohole im Sinne der vorliegenden Schrift sind solche Verbindungen, die mindestens eine N-(C=O)-N-Gruppe und mindestens eine Hydroxygruppe (-OH) aufweisen.

Typische Nebenprodukte der Umsetzung von ringförmigen oder offenkettigen N-hydroxyalkylierten Amiden, in denen Z¹ N-H ist, mit (Meth)acrylsäure oder (Meth)acrylsäureestern sind das über dieses Stickstoffatom verknüpfte (Meth)acrylamid, das über dieses Stickstoffatom verknüpfte Additionsprodukt an die Doppelbindung des Edukt-(Meth)acrylats bzw. des Ureido-(Meth)acrylats, das über die freie Hydroxygruppe verknüpfte Additionsprodukt an die Doppelbindung des Edukt-(Meth)acrylats bzw. des Ureido-(Meth)acrylats, sowie Produkte, die aus einer intra- oder intermolekularen Abspaltung der Gruppe R⁵-Z¹ aus den ringförmigen oder offenkettigen N-hydroxyalkylierten Amiden resultieren, die dann wiederum ver- oder umgeestert werden können.

Die katalysierte Ver- oder Umesterung erfolgt im Allgemeinen bei einer Temperatur des Reaktionsgemisches von 30 bis 140 °C, bevorzugt bei 50 bis 120 °C, besonders bevorzugt bei 60 bis 100°C und ganz besonders bevorzugt bei 60 bis 90 °C.

Gegebenenfalls kann die Reaktion unter leichtem Vakuum von beispielsweise 300 mbar bis Normaldruck durchgeführt werden, wenn das bei der Veresterung freigesetzte Wasser oder der bei der Umesterung entstehende niedrigsiedende Alkohol, gegebenenfalls als Azeotrop, abdestilliert werden soll.

Das molare Verhältnis zwischen (Meth)acrylsäure oder (Meth)acrylsäureester und Alkohol (C) oder (O) beträgt bei der durch ein anorganisches Salz katalysierten Ver- oder Umesterung in Gegenwart von erfindungsgemäß bestimmter Menge von Wasser in der Regel 1 bis 30:1 mol/mol, bevorzugt 1,5 bis 20:1 mol/mol und besonders bevorzugt 2 bis 10:1 mol/mol.

Der Gehalt an anorganischen Salzen im Reaktionsgemisch liegt in der Regel im Bereich von etwa 0,01 bis 5 mol-%, bevorzugt 0,1 bis 1,8 und besonders bevorzugt 0,5 bis 1,5 mol-% bezogen auf die Summe der eingesetzten Komponenten (C) beziehungsweise (O).

Bei der Ver- oder Umesterung sind gegebenenfalls Polymerisationsinhibitoren erforderlich. Bei der erfindungsgemäßen Reaktionsführung kann der Reaktionsmischung über den ohnehin in dem (Meth)acrylsäureester enthaltenen Polymerisationsinhibitoren hinaus zusätzliche Polymerisationsinhibitoren zugegeben werden, beispielsweise Hydrochinonmonomethylether, Phenothiazin, Phenole, wie z.B. 2-tert-Butyl-4-methylphenol, 6-tert.-Butyl-2,4-dimethylphenol oder N-Oxyle, wie 4-Hydroxy-2,2,6,6-tetramethylpiperidin-N-oxyl, 4-Oxo-2,2,6,6-tetramethylpiperidin-N-oxyl oder Uvinul^{®} 4040P der BASF SE oder Amine wie Kerobit^{®} BPD der BASF SE (N,N'-di-sec.-Butyl-p-phenylendiamin), beispielsweise in Mengen von 50 bis 2000 ppm.

Die Anwesenheit von sauerstoffhaltigen Gasen während der durch ein anorganisches Salz katalysierten Reaktion ist bevorzugt. Vorteilhaft wird die Ver- oder Umesterung in Gegenwart eines sauerstoffhaltigen Gases, bevorzugt Luft oder Luft-StickstoffGemische, durchgeführt.

Bei der katalysierten Ver- oder Umesterung werden in der Regel die Produkte mit einer Farbzahl (gemäß DIN ISO 53409) unter 250 Hazen, bevorzugt unter 150 und besonders bevorzugt unter 60 erhalten.

Die Reaktion kann in organischen Lösungsmitteln oder deren Gemischen oder ohne Zusatz von Lösungsmitteln ablaufen. Die Ansätze können bis auf das erfindungsgemäß zugegebene Wasser weitgehend wasserfrei sein. Alternativ können die Ansätze einen Wassergehalt aufweisen, der bei der Zugabe des Wassers berücksichtigt werden kann und gegebenenfalls bei zu hohem Wassergehalt azeotrop abgesenkt werden kann.

Ferner sind die Lösemittel weitgehend frei von primären und sekundären Alkoholen, d.h. unter 10 Gew.-% Alkoholgehalt, bevorzugt unter 5 Gew.-% Alkoholgehalt, besonders bevorzugt unter 1 Gew.-% Alkoholgehalt und ganz besonders bevorzugt unter 0,5 Gew.-% Alkoholgehalt.

Geeignete organische Lösungsmittel sind solche für diese Zwecke bekannten, beispielsweise tertiäre Monoole, wie C₃-C₆-Alkohole, bevorzugt tert-Butanol, tert-Amylalkohol, Pyridin, Poly-C₁-C₄-alkylenglykoldi-C₁-C₄-alkylether, bevorzugt Polyethylenglykoldi-C₁-C₄-alkylether, wie z.B. 1,2-Dimethoxyethan, Diethylenglykoldimethylether, Polyethylenglykoldimethylether 500, C₁-C₄-Alkylencarbonate, insbesondere Propylencarbonat, Essigsäure-C₃-C₆-alkylester, insbesondere Essigsäure-tert-butylester, Tetrahydrofuran (THF), Toluol, 1,3-Dioxolan, Aceton, iso-Butylmethylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen.

In einer besonders bevorzugten Ausführungsform der Umesterung wird die Reaktion in dem als Edukt eingesetzten (Meth)acrylsäureester durchgeführt. Ganz besonders bevorzugt ist die Durchführung der Reaktion in der Weise, dass das Produkt (C) oder (O) nach Beendigung der Reaktion als etwa 10 bis 80 gew.-%ige Lösung in dem als Edukt eingesetzten (Meth)acrylsäureester anfällt, insbesondere als 20 bis 50 gew.-%ige Lösung.

Die Edukte liegen entweder gelöst, als Feststoffe suspendiert oder in Emulsion im Reaktionsgemisch vor. Vorzugsweise liegt die anfängliche Konzentration der Reaktanden im Bereich von etwa 0,1 bis 20 mol/l, insbesondere bei 0,15 bis 10 mol/l oder 0,2 bis 5 mol/l.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Geeignete Reaktoren sind dem Fachmann bekannt. Bevorzugt erfolgt die Umsetzung in einem Rührkesselreaktor oder einem Festbettreaktor.

Zur Durchmischung des Ansatzes können beliebige Verfahren eingesetzt werden. Spezielle Rührvorrichtungen sind nicht erforderlich. Die Durchmischung kann beispielsweise durch Einspeisen eines Gases, bevorzugt eines sauerstoffhaltigen Gases (siehe unten) erfolgen. Das Reaktionsgemisch kann ein- oder mehrphasig sein und die Reaktanden werden darin gelöst, suspendiert oder emulgiert vorgelegt. Die Temperatur wird während der Reaktion auf den gewünschten Wert eingestellt und kann, falls gewünscht, während des Reaktionsverlaufs erhöht oder verringert werden.

Die Entfernung von Wasser im Falle einer Veresterung oder Alkoholen, die bei einer Umesterung aus den Alkyl(meth)acrylaten freigesetzt werden, erfolgt kontinuierlich oder schrittweise in an sich bekannter Weise, z.B. Destillation unter Normaldruck oder azeotrope Entfernung, Strippen, Adsorption, Pervaporation und Diffusion über Membranen oder Extraktion.

Das Strippen kann beispielsweise durch Durchleiten eines sauerstoffhaltigen Gases, bevorzugt eines Luft- oder Luft-Stickstoff-Gemisches, durch das Reaktionsgemisch erfolgen, gegebenenfalls zusätzlich zu einer Destillation.

Zur Absorption eignen sich vorzugsweise Molekularsiebe oder Zeolithe mir einer Porengröße z.B. im Bereich von etwa 3 bis 10 Å, eine Abtrennung durch Destillation oder mit Hilfe geeigneter semipermeabler Membranen.

Es ist aber auch möglich, das abgetrennte Gemisch aus Alkyl(meth)acrylat und dem diesem zugrundeliegenden Alkohol, das häufig ein Azeotrop bildet, direkt in eine Anlage zur Herstellung des Alkyl(meth)acrylats zuzuführen, um es dort in einer Veresterung mit (Meth)acrylsäure wieder zu verwerten.

Nach Beendigung der Reaktion kann man das erhaltene Produktgemisch ohne weitere Aufreinigung weiterverwenden oder es erforderlichenfalls in einem weiteren Schritt aufreinigen.

In der Regel wird lediglich der eingesetzte heterogene Katalysator vom Produktgemisch abgetrennt und das gewünschte Reaktionsprodukt vom gegebenenfalls verwendeten organischen Lösungsmittel abgetrennt.

Eine Abtrennung vom heterogenen Katalysator erfolgt in der Regel durch Filtration, Elektrofiltration, Absorption, Zentrifugation oder Dekantieren, vorzugsweise durch Filtration. Der abgetrennte heterogene Katalysator kann anschließend für weitere Reaktionen eingesetzt werden.

Die Abtrennung vom organischen Lösungsmittel erfolgt in der Regel durch Destillation, Rektifikation oder bei festen Reaktionsprodukten durch Filtration.

Zur weiteren Aufreinigung des Reaktionsproduktes kann auch eine Chromatographie durchgeführt werden.

Bevorzugt werden jedoch lediglich der heterogene Katalysator und das gegebenenfalls eingesetzte Lösungsmittel abgetrennt. Die Reaktionsbedingungen bei der erfindungsgemäßen Ver- oder Umesterung sind vorzugsweise so gewählt, dass die Bildung von Nebenprodukten reduziert wird, die zum Beispiel von unerwünschter radikalischer Polymerisation des eingesetzten (Meth)acrylats stammen und sonst nur durch Zugabe von Stabilisatoren verhindert werden kann.

Die erfindungsgemäß hergestellten (Meth)acrylsäureester finden Anwendungen beispielsweise als Monomere oder Comonomere in der Herstellung von Dispersionen, beispielsweise Acryldispersionen, als Reaktivverdünner, beispielsweise in strahlungshärtbaren Beschichtungsmassen oder in Farben, bevorzugt in Aussenfarben, sowie in Dispersionen für Anwendung im Papierbereich, im Kosmetikbereich, im Pharmabereich, in Agroformulierungen, in der Textilindustrie und im Bereich der Ölförderung.

Die folgenden Beispiele sollen die Eigenschaften der Erfindung erläutern, ohne sie aber einzuschränken.

### Beispiele

Die Versuche wurden für insgesamt vier unterschiedliche Zusammensetzungen des heterogenen Katalysators K₃PO₄ (K1, K2, K3, K4) durchgeführt.

Die Katalysatoren K1, K2, K3 und K4 wurden mittels Elementaranalyse analysiert und die Ergebnisse für deren Zusammensetzungen sind in Tabelle 1 aufgelistet, wobei die Elemente in dem Katalysator als Salz gebunden sind.

**Tabelle 1: Zusammensetzung der Katalysatoren K1 bis K4 bestimmt mittels Elementaranalyse**

| | Cl⁻ | S²⁻ | Al³⁺ | Fe^{2+/3+} | K⁺ | Li⁺ | Na⁺ | P |
|---|---|---|---|---|---|---|---|---|
| | mg/kg | mg/kg | mg/kg | mg/kg | g/100 g | mg/kg | g/100 g | g/100 g |
| K1 | 36 | 25 | <3 | 4 | 52 | <3 | 0,3 | 14 |
| K2 | 34 | 47 | <3 | 4 | 53 | <3 | 0,5 | 14 |
| K3 | 25 | 52 | <3 | 29 | 54 | <3 | 0,23 | 14,4 |
| K4 | 98 | <2 | <3 | 9 | 52 | <3 | 0.26 | 13.5 |

Zusätzlich wurde die Partikelgröße der K₃PO₄ Katalysatoren K1, K2 und K3 mittels Laserbeugung (Malvern Mastersize 2000) und der Dispergiereinheit Scirocco bei Drücken von 0,5 bar, 1,0 bar und 3,0 bar bestimmt. Die Ergebnisse sind in Tabelle 2 wiedergegeben.

**Tabelle 2: Partikelgröße der Katalysatoren K1 bis K3 bestimmt mittels Laserbeugung.**

| | 1 bis 10 µm | 10 bis 100 µm | 100 bis 1000 µm | > 1000 µm |
|---|---|---|---|---|
| K1 | 1 % | 30 % | 70 % | 0 |
| K2 | 9 % | 53 % | 38 % | 0 |
| K3 | 2 % | 33 % | 55 % | 10 % |

### Beispiel 1: Umesterung von MMA mit 800 ppm Wasser

Die Umesterung erfolgte in einem 750 mL-Doppelmantelreaktor ausgestattet mit einem Anker-Rührer, einer Trennkolonne und einem Flüssigkeitsteiler. Das Rücklaufverhältnis betrug 3:1 (Rücklauf : Ablauf), die Rührgeschwindigkeit (Anker-Rührer) 200-250 U/min und die Lufteinleitung 1,5 L/h.

In dieser Apparatur wurden 0,041 g Phenothiazin (PTZ), 0,287 g Methylhydrochinon (MEHQ) (Polymerisationsinhibitoren) und 690 g Methylmethacrylat (MMA) bei Raumtemperatur vorgelegt. Das Reaktionsgemisch wurde aufgeheizt und 130 g 2-Hydroxyethylethylenharnstoff (HEEH) zugegeben, dann wurde weiter aufgeheizt und der Katalysator (K₃PO₄, 2,65 g) sowie 800 ppm Wasser zugegeben. Anschließend wurde ein Druck von 450 mbar (abs) eingestellt und die Sumpftemperatur ergab sich zu 73 bis 80°C.

Die Versuche wurden für die Katalysatoren K1 bis K3 jeweils mit einem Zusatz von 800 ppm Wasser durchgeführt. Nach 5 Stunden Versuchsdauer wurde die Destillation abgestellt und das Sumpfprodukt gaschromatographisch (GC) und hochleistungsflüssigkeitschromatographisch (HPLC) auf das Produkt Ureidomethacrylat (UMA) sowie nicht umgesetztes HEEH, Nebenprodukt I und Nebenprodukt II untersucht.

Die aufgefundenen Nebenkomponenten sind u.a. das Bisacrylat entstanden durch Bildung des Amids durch Reaktion von UMA mit einem weiteren Äquivalent Methylmethacrylat (Nebenprodukt I) und das Additionsprodukt des eingesetzten Alkohols HEEH an die Doppelbindung des gewünschten Produkts (Nebenprodukt II).

Die Ergebnisse sind in Tabelle 3 dargestellt.

**Tabelle 3: Ergebnisse der Umesterung von MMA mit HEEH zu UMA in Gegenwart der Katalysatoren K1, K2 oder K3, wobei der Reaktion 800 ppm Wasser zugeführt wurden.**

| K₃PO₄ | Zusatz Wasser (ppm) | HEEH [GC%] | Nebenprodukt I [GC%] | Nebenprodukt II [GC%] | UMA [GC%] |
|---|---|---|---|---|---|
| K1 | 800 | 0 | 0,4 | 2,3 | 17,9 |
| K2 | 800 | 0 | 0,5 | 1,8 | 18,9 |
| K3 | 800 | 0 | 0,4 | 1,9 | 19,4 |

### Vergleichsbeispiel 1: Umesterung von MMA ohne Wasser

Zum Vergleich wurden die Versuche für die Katalysatoren K1 bis K3 wie in Beispiel 1 beschrieben ohne die Zugabe von Wasser durchgeführt. Die Ergebnisse der Vergleichsversuche sind in Tabelle 4 gelistet.

**Tabelle 4: Ergebnisse der Umesterung von MMA mit HEEH zu UMA in Gegenwart der Katalysatoren K1, K2 oder K3, wobei der Reaktion kein zusätzliches Wasser zugeführt wurde.**

| K₃PO₄ | Zusatz Wasser (ppm) | HEEH [GC%] | Nebenprodukt I [GC%] | Nebenprodukt II [GC%] | UMA [GC%] |
|---|---|---|---|---|---|
| K1 | 0 | 0 | 0,4 | 5,1 | 15 |
| K2 | 0 | 0 | 0,9 | 6,8 | 15,9 |
| K3 | 0 | 0 | 0,8 | 9,2 | 14,3 |

Der Vergleich der Versuche aus Beispiel 1 und Vergleichsbeispiel 1 zeigt eindeutig, dass der gezielte Zusatz von Wasser gegenüber den im Stand der Technik bekannten Verfahren in einer verbesserten Ausbeute resultiert. Mit einem geringen Anteil des Vernetzers und der Nebenprodukte verläuft die Umesterung mit hoher Selektivität und hohem Umsatz.

### Beispiel 2: Umesterung von MMA mit 800 ppm Wasser

Die Versuche wurden für die Katalysatoren K1 und K3 in einem beheizbaren Doppelmantelreaktor (Fassungsvermögen 4L) mit einem Ankerrührer, einer Trennkolonne und einem Flüssigkeitsteiler durchgeführt. Die Rührgeschwindigkeit (Anker-Rührer) betrug 160 U/min und die Lufteinleitung 1,5 L/h.

In dieser Apparatur wurden 0,123 g Phenothiazin (PTZ), 0,861 g Methylhydrochinon (MEHQ) (Polymerisationsinhibitoren) und 2070 g MMA bei Raumtemperatur vorgelegt. Das Reaktionsgemisch wurde auf 70 °C aufgeheizt und 390 g 2-Hydroxyethylethylenharnstoff (HEEH), der Katalysator (K₃PO₄, 7,95 g) sowie 800 ppm Wasser zugegeben. Der so erhaltenen Ansatz umfasste insgesamt 50 ppm PTZ, 350 ppm MEHQ, 20,7 mol MMA, 3 mol HEEH, 800 ppm Wasser und 1,25 mol-% (bezogen auf HEEH) K₃PO₄. Anschließend wurde ein Vakuum von 450 mbar eingestellt und die Sumpftemperatur wurde auf 78 bis 79 °C erhöht, bis die Destillation unter vollem Rückfluss startete. Nach 30 min wurde kontinuierlich Destillat teilweise zurückgeführt (Rücklauf/Verhältnis 3:1).

Nach 5 Stunden Versuchsdauer wurde die Destillation abgestellt und der Sumpf mittels GC und HPLC auf das Produkt Ureidomethacrylat (UMA) sowie entstandene Nebenkomponenten untersucht. Die Ergebnisse dieser Versuchsreihe sind in Tabelle 5 aufgelistet.

**Tabelle 5: Ergebnisse der Umesterung von MMA mit HEEH zu UMA in einem 4L Reaktor, wobei der Reaktion 800 ppm Wasser zugeführt wurde.**

| K₃PO₄ | Zusatz Wasser [ppm] | HEEH [HPLC%] | Nebenprodukt I [HPLC%] | Nebenprodukt II [HPLC%] | UMA [HPLC%] |
|---|---|---|---|---|---|
| K1 | 800 | 5,6 | 0,06 | 0,19 | 15,47 |
| K3 | 800 | 6,02 | 0,06 | 0,18 | 15,13 |

### Vergleichsbeispiel 2: Umesterung von MMA ohne Wasser

Zum Vergleich wurden die Versuche für die Katalysatoren K1 und K3 wie in Beispiel 2 beschrieben ohne die Zugabe von Wasser durchgeführt. Die Ergebnisse der Vergleichsversuche sind in Tabelle 6 dargestellt.

**Tabelle 6: Ergebnisse der Umesterung von MMA mit HEEH zu UMA in einem 4L Reaktor, wobei der Reaktion kein Wasser zugeführt wurde.**

| K₃PO₄ | Zusatz Wasser [ppm] | HEEH [HPLC%] | Nebenprodukt I [HPLC%] | Nebenprodukt II [HPLC%] | UMA [HPLC%] |
|---|---|---|---|---|---|
| K1 | 0 | 1,73 | 0,23 | 1,02 | 20,61 |
| K3 | 0 | 1,61 | 0,26 | 1,56 | 19,67 |

Der Vergleich der Versuche zeigt eindeutig, dass der gezielte Zusatz von Wasser den Anteil der Nebenprodukte zurückdrängt und die Umesterung mit hoher Selektivität verläuft.

### Beispiel 3 Umesterung von MMA in Abhängigkeit vom Wassergehalt

Für die Katalysatoren K1, K2, K3 und K4 wurde eine Messreihe in Abhängigkeit vom Wassergehalt durchgeführt. Dazu wurde ein 4L- Doppelmantelreaktor ausgestattet mit einem Anker-Rührer, einer Trennkolonne und einem Flüssigkeitsteiler verwendet. Das Rücklaufverhältnis betrug 3:1 (Rücklauf : Ablauf), die Rührgeschwindigkeit (Anker-Rührer)160 U/min und die Lufteinleitung 1,5 L/h.

In dieser Apparatur wurden wie in Beispiel 2 beschrieben PTZ, MEHQ und MMA bei Raumtemperatur vorgelegt und HEEH, K₃PO₄ sowie jeweils unterschiedliche Wassermengen im Bereich von 0 bis 2000 ppm hinzugegeben.

Anschließend wurde der Ansatz unter Luftleitung auf 95°C Manteltemperatur erwärmt sowie ein Vakuum von 450 mbar und ein Rücklaufverhältnis von 3:1 eingestellt. Das entstehende Azeotrop aus MeOH sowie MMA wurde abdestiliert.

Nach sechs Stunden Versuchsdauer wurde die Destillation abgestellt und der Sumpf mittels GC und HPLC auf das Produkt Ureidomethacrylat (UMA) sowie entstandene Nebenkomponenten untersucht. Die aufgefundenen Nebenkomponenten sind u.a. das Bisacrylat entstanden durch Bildung des Amids durch Reaktion von UMA mit einem weiteren Äquivalent Methylmethacrylat (Nebenprodukt I) und das Additionsprodukt des eingesetzten Alkohols HEEH an die Doppelbindung des Produkts (Nebenprodukt II).

Um die Abhängigkeit von den Katalysatorzusammensetzungen und der Wassermenge aufzuzeigen, wurden die Versuche für vier unterschiedliche Katalysatoren K1, K2, K3 und K4, wie oben charakterisiert, mit jeweils unterschiedliche Mengen von Wasser im Bereich von 0 bis 2000 ppm durchgeführt.

Die Ergebnisse nach 6 Stunden sind für die vier Katalysatoren K1, K2, K3 und K4 in Abhängigkeit von der zugegebenen Wassermenge in Tabelle 7 dargestellt. In Tabelle 7 ist weiterhin für jeden Wassergehalt der Anteil an HEEH und UMA im Produkt aufgelistet. Zusätzlich zu den Nebenprodukten und dem Vernetzter sind in Tabelle 7 der Anteil an umgesetztem HEEH (in %), der Kalium-Gehalt (in ppm) und die Farbzahl (in Hazen) enthalten.

Die Ergebnisse der Tabelle 7 zeigen deutlich, dass die Zugabe von Wasser im Bereich von 500 bis 1500 ppm für jeden Katalysator K1 bis K4 zu einem erhöhten Umsatz an UMA führt. Auch der Anteil an Vernetzter und Nebenprodukten sowie der Kaliumgehalt sind in diesem Bereich verringert. Zusätzlich zeigen die Versuche, dass das erfindungsgemäß vorgeschlagene Verfahren die Herstellung von hellen Produkten mit Farbzahlen zwischen 40 und 65 Hazen ermöglicht.

**Tabelle 7 : Ergebnisse der Umesterung von MMA mit HEEH zu UMA für die vier Katalysatoren K1, K2, K3 und K4 in Abhängigkeit von der Wasserzugabe.**

| # | K₃PO₄ | H₂O-Zugabe [ppm] | HEEH 6h [HPLC%] | UMA 6h [HPLC%] | Umsatz HEEH 6h [%] | Nebenprodukt I 6 h [%] | Nebenprodukt II 6 h [%] | K-Gehalt [ppm] | Farbzahl Hazen |
|---|---|---|---|---|---|---|---|---|---|
| **Katalysator 1** | | | | | | | | | |
| 1 | K1 | 0 | 0,3 | 28,1 | 98,9 | 3,5 | 1,1 | 180 | 104 |
| 2 | K1 | 500 | 0,4 | 29,7 | 98,7 | 3,1 | 1,1 | 190 | 81 |
| 3 | K1 | 1000 | 1,2 | 30,1 | 96,2 | 2 | 0,5 | 225 | 50 |
| 4 | K1 | 1500 | 0,2 | 27,7 | 99,3 | 3 | 0,9 | 140 | 220 |
| 5 | K1 | 2000 | 4 | 27,1 | 87,1 | 0,8 | 0,2 | 320 | 53 |

| **Katalysator 2** | | | | | | | | | |
|---|---|---|---|---|---|---|---|---|---|
| 6 | K2 | 0 | 0,2 | 26,6 | 99,3 | 4,4 | 1,5 | 150 | 65 |
| 7 | K2 | 500 | 1,9 | 30,9 | 94,2 | 1,7 | 0,4 | 165 | 40 |
| 8 | K2 | 1000 | 1,9 | 28 | 93,6 | 1,4 | 0,4 | 200 | 47 |
| 9 | K2 | 1500 | 1,5 | 30,7 | 95,3 | 1,8 | 0,5 | 205 | 76 |
| 10 | K2 | 2000 | 1,8 | 29,6 | 94,3 | 1,6 | 0,5 | 235 | 126 |

| **Katalysator 3** | | | | | | | | | |
|---|---|---|---|---|---|---|---|---|---|
| 11 | K3 | 0 | 0,1 | 24,7 | 99,6 | 7,6 | 2,2 | 165 | 116 |
| 12 | K3 | 1000 | 0,4 | 30,4 | 98,7 | 3,4 | 1,1 | 165 | 63 |
| 13 | K3 | 2000 | 2,6 | 29,8 | 92,0 | 1,2 | 0,3 | 240 | 58 |

| **Katalysator 4** | | | | | | | | | |
|---|---|---|---|---|---|---|---|---|---|
| 14 | K4 | 0 | 0,1 | 26,4 | 99,6 | 5,5 | 1,6 | 140 | 49 |
| 15 | K4 | 1000 | 1,0 | 28,6 | 96,6 | 2 | 0,6 | 165 | 52 |
| 16 | K4 | 2000 | 4,5 | 25,8 | 85,1 | 0,8 | 0,2 | 290 | 76 |

## Patentansprüche

1. Verfahren zur Herstellung von (Meth)acrylsäureestern durch Veresterung von (Meth)acrylsäure oder Umesterung von mindestens einem (Meth)acryisäureester mit mindestens einer Verbindung, die mindestens eine OH-Gruppe enthält, in Gegenwart eines heterogenen Katalysators, der mindestens ein anorganisches Salz ausgewählt aus der Gruppe bestehend aus K₃PO₄, Na₃PO₄, K₂CO₃ und Na₂CO₃ sowie deren Hydrate enthält, **dadurch gekennzeichnet, dass** die Veresterung oder Umesterung in Gegenwart von 300 bis 3000 ppm Wasser bezogen auf das Gesamtgewicht des Reaktionsgemisches durchgeführt wird.

2. Verfahren gemäß Anspruch 1, **dadurch gekennzeichnet, dass** die Veresterung oder Umesterung in Gegenwart von 400 bis 2000 ppm Wasser bezogen auf das Gesamtgewicht des Reaktionsgemisches durchgeführt wird.

3. Verfahren gemäß einem der vorhergehenden Ansprüche, **dadurch gekennzeichnet, dass** die Veresterung oder Umesterung in Gegenwart von 500 bis 1200 ppm Wasser bezogen auf das Gesamtgewicht des Reaktionsgemisches durchgeführt wird.

4. Verfahren gemäß einem der vorhergehenden Ansprüche, **dadurch gekennzeichnet, dass** das Wasser bei einem Ansetzen eines Reaktionsgemisches in einem Verfahrensschritt vollständig oder in Teilen in mehreren Verfahrensschritten zugefügt wird.

5. Verfahren gemäß einem der vorhergehenden Ansprüche, **dadurch gekennzeichnet, dass** das Wasser vollständig in einem Verfahrensschritt oder in Teilen in mehreren Verfahrensschritten bei dem Ansetzen des Reaktionsgemisches vor oder nach Zugabe des heterogenen Katalysators zugeführt wird.

6. Verfahren gemäß einem der vorhergehenden Ansprüche, **dadurch gekennzeichnet, dass** das Wasser vollständig in einem Verfahrensschritt vor der Zugabe des heterogenen Katalysators zugeführt wird.

7. Verfahren gemäß Anspruch 6, **dadurch gekennzeichnet, dass** die Zugabe des Wassers vor der Zugabe der mindestens eine OH-Gruppe enthaltenden Verbindung erfolgt.

8. Verfahren gemäß einem der vorhergehenden Ansprüche, **dadurch gekennzeichnet, dass** der heterogene Katalysator wasserfrei oder hygroskopisch ist.

9. Verfahren gemäß einem der vorhergehenden Ansprüche, **dadurch gekennzeichnet, dass** die mindestens eine OH-Gruppe enthaltende Verbindung offenkettige oder ringförmige N-hydroxyalkylierte Amide enthält.

10. Verfahren gemäß einem der vorhergehenden Ansprüche, **dadurch gekennzeichnet, dass** das mindestens eine anorganische Salz einen pK_{B}-Wert von nicht mehr als 7,0 und nicht weniger als 1,0 sowie eine Löslichkeit im Reaktionsmedium bei 25 °C von nicht mehr als 1 g/l aufweist.

11. Verfahren gemäß einem der Ansprüche 1 bis 3, **dadurch gekennzeichnet, dass** das mindestens eine anorganische Salz enthält:
- mindestens ein Anion ausgewählt aus Carbonat, Hydrogencarbonat, Phosphat, Hydrogenphosphat, Dihydrogenphosphat, Sulfat, Sulfit und Carboxylat der Formel R⁶-COO⁻, worin R⁶ C₁-C₁₈-Alkyl oder gegebenenfalls durch ein oder mehrere Sauerstoff und/oder Schwefelatome und/oder ein oder mehrere substituierte oder unsubstituierte Iminogruppen unterbrochenes C₂-C₁₈- Alkyl oder C₆-C₁₂-Aryl bedeutet, und
- mindestens ein Kation ausgewählt aus Alkalimetallen, Erdalkalimetallen, Ammonium, Cer, Eisen, Mangan, Chrom, Molybdän, Kobalt, Nickel oder Zink.

12. Verfahren nach einem der vorstehenden Ansprüche, **dadurch gekennzeichnet, dass** die Reaktion in der Weise durchführt wird, dass das Produkt nach Beendigung der Reaktion als 10 bis 80 gew.-%ige Lösung in dem als Edukt eingesetzten (Meth)acrylsäureester anfällt.

## Claims

1. A process for preparing (meth)acrylic esters by esterifying (meth)acrylic acid or transesterifying at least one (meth)acrylic ester with at least one compound comprising at least one OH group in the presence of a heterogeneous catalyst comprising at least one inorganic salt selected from the group consisting of K₃PO₄, Na₃PO₄, K₂CO₃ and Na₂CO₃, and the hydrates thereof, which comprises performing the esterification or transesterification in the presence of 300 to 3000 ppm of water based on the total weight of the reaction mixture.

2. The process according to claim 1, wherein the esterification or transesterification is performed in the presence of 400 to 2000 ppm of water based on the to-tal weight of the reaction mixture.

3. The process according to either of the preceding claims, wherein the esterification or transesterification is performed in the presence of 500 to 1200 ppm of water based on the total weight of the reaction mixture.

4. The process according to any of the preceding claims, wherein the water, in the case that a reaction mixture is made up in one process step, is added completely or in portions in several process steps.

5. The process according to any of the preceding claims, wherein the water is supplied completely in one process step or in portions in several process steps when the reaction mixture is made up, before or after addition of the heterogeneous catalyst.

6. The process according to any of the preceding claims, wherein the water is supplied completely in one process step before the addition of the heterogeneous catalyst.

7. The process according to claim 6, wherein the addition of the water precedes the addition of the compound comprising at least one OH group.

8. The process according to any of the preceding claims, wherein the heterogeneous catalyst is anhydrous or hygroscopic.

9. The process according to any of the preceding claims, wherein the compound comprising at least one OH group comprises open-chain or cyclic N-hydroxyalkylated amides.

10. The process according to any of the preceding claims, wherein the at least one inorganic salt has a pK_{B} of not more than 7.0 and not less than 1.0, and a solubility in the reaction medium at 25°C of not more than 1 g/l.

11. The process according to any of claims 1 to 3, wherein the at least one inorganic salt comprises:
- at least one anion selected from carbonate, hydrogencarbonate, phosphate, hydrogenphosphate, dihydrogenphosphate, sulfate, sulfite and carboxylate of the formula R⁶-COO- in which R⁶ is C₁-C₁₈-alkyl, or C₂-C₁₈- alkyl optionally interrupted by one or more oxygen and/or sulfur atoms and/or one or more substituted or unsubstituted imino groups, or C₆-C₁₂-aryl, and
- at least one cation selected from alkali metals, alkaline earth metals, ammonium, cerium, iron, manganese, chromium, molybdenum, cobalt, nickel and zinc.

12. The process according to any of the preceding claims, wherein the reaction is performed in such a way that the product, after the reaction has ended, is obtained as a 10 to 80% by weight solution in the (meth)acrylic ester used as the reactant.

## Revendications

1. Procédé pour la préparation d'esters de l'acide (méth)acrylique) par estérification d'acide (méth)acrylique ou transestérification d'au moins un ester de l'acide (méth)acrylique avec au moins un composé, qui contient au moins un groupe OH, en présence d'un catalyseur hétérogène, qui contient au moins un sel inorganique choisi dans le groupe constitué par K₃PO₄, Na₃PO₄, K₂CO₃ et Na₂CO₃ ainsi que leurs hydrates, **caractérisé en ce que** l'estérification ou la transestérification est réalisée en présence de 300 à 3000 ppm d'eau, par rapport au poids total du mélange réactionnel.

2. Procédé selon la revendication 1, **caractérisé en ce que** l'estérification ou la transestérification est réalisée en présence de 400 à 2000 ppm d'eau, par rapport au poids total du mélange réactionnel.

3. Procédé selon l'une quelconque des revendications précédentes, **caractérisé en ce que** l'estérification ou la transestérification est réalisée en présence de 500 à 1200 ppm d'eau, par rapport au poids total du mélange réactionnel.

4. Procédé selon l'une quelconque des revendications précédentes, **caractérisé en ce que** l'eau est ajoutée complètement dans une étape de réaction ou en parties dans plusieurs étapes de réaction, lors de la préparation d'un mélange réactionnel.

5. Procédé selon l'une quelconque des revendications précédentes, **caractérisé en ce que** l'eau est ajoutée complètement dans une étape de réaction ou en parties dans plusieurs étapes de réaction lors de la préparation du mélange réactionnel, avant ou après l'addition du catalyseur hétérogène.

6. Procédé selon l'une quelconque des revendications précédentes, **caractérisé en ce que** l'eau est ajoutée complètement dans une étape de réaction avant l'addition du catalyseur hétérogène.

7. Procédé selon la revendication 6, **caractérisé en ce que** l'addition de l'eau est réalisée avant l'addition du composé contenant au moins un groupe OH.

8. Procédé selon l'une quelconque des revendications précédentes, **caractérisé en ce que** le catalyseur hétérogène est anhydre ou hygroscopique.

9. Procédé selon l'une quelconque des revendications précédentes, **caractérisé en ce que** le composé contenant au moins un groupe OH contient des amides N-hydroxyalkylés à chaîne ouverte ou cycliques.

10. Procédé selon l'une quelconque des revendications précédentes, **caractérisé en ce que** ledit au moins un sel inorganique présente une valeur pK_{B} qui n'est pas supérieure à 7,0 et pas inférieure à 1,0, ainsi qu'une solubilité dans le milieu réactionnel à 25°C qui n'est pas supérieure à 1 g/1.

11. Procédé selon l'une quelconque des revendications 1 à 3, **caractérisé en ce que** ledit au moins un sel inorganique contient :
- au moins un anion choisi dans parmi le carbonate, l'hydrogénocarbonate, le phosphate, l'hydrogénophosphate, le dihydrogénophosphate, le sulfate, le sulfite et le carboxylate de formule R⁶COO-, R⁶ signifiant C₁-C₁₈-alkyle ; ou C₂-C₁₈-alkyle ou C₆-C₁₂-aryle le cas échéant interrompu par un ou plusieurs atomes d'oxygène et/ou de soufre et/ou par un ou plusieurs groupes imino substitués ou non substitués, et
- au moins un cation choisi parmi les métaux alcalins, les métaux alcalino-terreux, l'ammonium, le cérium, le fer, le manganèse, le chrome, le molybdène, le cobalt, le nickel ou le zinc.

12. Procédé selon l'une quelconque des revendications précédentes, **caractérisé en ce que** la réaction est réalisée de manière telle que le produit, après la fin de la réaction, est obtenu sous forme d'une solution à environ 10-80% en poids dans l'ester de l'acide (méth)acrylique utilisé comme produit de départ.
